# EUROPEAN PATENT SPECIFICATION

(11) **EP 2 089 093 B1**
(45) Date of publication and mention of the grant of the patent: **29.08.2012**
(21) Application number: 07863972.1
(22) Date of filing: 06.11.2007
(51) Int. Cl.: A61M 31/00, A61M 39/04

(54) **VASCULAR ACCESS DEVICES INCLUDING A TEAR-RESISTANT SEPTUM**
GEFÄSSZUGANGSVORRICHTUNGEN MIT EINEM RISSFESTEN SEPTUM
DISPOSITIFS D'ACCÈS VASCULAIRE COMPORTANT UN SEPTUM RÉSISTANT À LA DÉCHIRURE

(30) Priority: 06.11.2006 US 864539 P; 05.11.2007 US 935097
(43) Date of publication of application: 19.08.2009
(73) Proprietor: Becton, Dickinson and Company, Franklin Lakes, NJ 07417 (US)
(72) Inventor: HARDING, Weston F., Lehi, Utah 84043 (US); KOMMIREDDY, Dinesh S., Midvale, Utah 84047 (US); STOUT, Marty L., South Weber, Utah 84405 (US); HILLMAN, Jason, West Jordan, Utah 84088 (US); RASMUSSEN, Wayne K., Riverdale, Utah 84405 (US); MOULTON, William, G., West Jordan, UT 84084 (US)
(74) Representative: von Kreisler Selting Werner
(86) International application number: PCT/US2007/083790
(87) International publication number: WO 2008/058135

(56) References cited:
- EP-A1- 1 236 482
- JP-A- 11 276 595
- US-A- 5 114 408
- US-A1- 2003 085 373
- US-A1- 2005 256 500
- US-A1- 2005 267 487
- US-B1- 6 610 031
- US-B1- 6 623 460

## Description

### BACKGROUND OF THE INVENTION

The present disclosure relates to infusion therapy with vascular access devices. Infusion therapy is one of the most common health care procedures. Hospitalized, home care, and other patients receive fluids, pharmaceuticals, and blood products via a vascular access device inserted into the vascular system. Infusion therapy may be used to treat an infection, provide anesthesia or analgesia, provide nutritional support, treat cancerous growths, maintain blood pressure and heart rhythm, or many other clinically significant uses.

Infusion therapy is facilitated by a vascular access device. The vascular access device may access a patient's peripheral or central vasculature. The vascular access device may be indwelling for short term (days), moderate term (weeks), or long term (months to years). The vascular access device may be used for continuous infusion therapy or for intermittent therapy.

A common vascular access device is a plastic catheter that is inserted into a patient's vein. The catheter length may vary from a few centimeters for peripheral access to many centimeters for central access. The catheter may be inserted transcutaneously or may be surgically implanted beneath the patient's skin. The catheter, or any other vascular access device attached thereto, may have a single lumen or multiple lumens for infusion of many fluids simultaneously.

Vascular access devices commonly include a Luer adapter, or other connector or adapter, to which other medical devices may be attached. For example, an IV (intravenous) administration set may be attached to a vascular access device to provide a fluid conduit for the continuous infusion of fluids and pharmaceuticals from an intravenous (IV) bag. A variety of medical devices may cooperate with vascular access devices to provide selective, temporary, or long-term access to the vascular system of a patient. A vascular access device may include a body having a lumen through the body and a septum for selectively closing the lumen. The septum may be opened with a blunt cannula, a male Luer of a medical device, or other suitable medical device.

Vascular access devices provide many significant benefits to patients and medical practitioners. A vascular access device is most beneficial to patients when the septum forms a proper seal between the accessed vascular system and the outside or external environment. In an ideal vascular access device, the septum would continuously seal the patient's vascular system, which may include external vascular equipment intentionally coupled to the patient's internal vascular system by a medical practitioner, from the external environment.

As with most systems, one of the biggest challenges to the proper function of the vascular access device is when there is a change in the system, such as when different medical devices are connected or disconnected from the vascular access device. If the seal against the external environment is broken during the connection or disconnection of a medical device, there is the possibility of infection being introduced into the patient's vascular system. Additionally, if a pressure difference is created across the vascular access device, there becomes the possibility that blood will be drawn up the catheter system and possibly into the vascular access device or beyond. Alternatively, a pressure difference across the vascular access device may make it more difficult to couple other medical devices to the vascular access device.

As introduced above, vascular access devices are often coupled with a blunted cannula, such as the tip of a syringe, with a male Luer connector, or with other medical devices. These medical devices may be coupled to the vascular access devices by pressing a portion of the medical device into a slit or passage in the septum. Some medical devices are coupled to the vascular access device through a twisting motion by which the body or other portion of the medical device is coupled to the body of the vascular access device and by which a portion of the medical device is disposed in the slit or passage of the septum. Other methods of coupling the vascular access device to one or more medical devices may be used as well.

Regardless of the methods used to couple medical devices to the vascular access device, repeated transitions of the septum between open and closed configurations applies stress to the septum. In some experiences the septum has been seen to tear, either slightly or more significantly, at the edges of the slit that allows other devices to access the internal vascular system through the lumen of the body. In previous vascular access devices, two common tear patterns have been observed: radial tearing and circumferential tearing. Depending on the nature of the tear, the impacts of the tear may include a decrease in the quality of the seal formed by the septum or pieces or particles of the septum breaking free from the remainder of the septum. In any event, a septum that is modified from the manufacturer's intended and safety-tested design is not preferred for a number of reasons. The present disclosure is directed to vascular access devices, and methods of manufacturing vascular access devices, that include a tear-resistant septum.
A vascular access device having a body defining a lumen is, for example, described in US 2005/256500. To seal the lumen extending through the body, a septum is located in the body. The septum has a slit adapted to provide selective passage through the septum.

### BRIEF SUMMARY OF THE INVENTION

The above-mentioned and other problems are solved by a vascular access device according to claim 1 and a method of manufacturing a vascular access device according to claim 11 respectively.

A vascular access device includes a body and a septum. The body defines a lumen extending through the body. The septum is at least partially disposed in the body to at least substantially seal the lumen extending through the body. The septum has a longitudinal axis, has a slit adapted to provide selective passage through the septum, and is configured to resist tearing near the slit. The slit has a slit width between a slit first end and a slit second end, and extends orthogonally to the longitudinal axis of the septum.

The septum includes a top disk. The top disk may include a first cross section and a second cross section. The first cross section is parallel to the slit and is thinner than the second cross section. At least a portion of the slit within the top disk may have at least one relief cut. The at least one relief cut may be in line with the slit width and/or may be perpendicular to the slit width.

The first slit end and the second slit end may each terminate at a hole within the top disk. The first slit end and the second slit end may each travel in a direction opposite the direction that a separate vascular access device would rotate when accessing the septum.

The slit width may extend to the full width of the septum at the top disk. The slit depth may extend in the direction of the longitudinal axis and the slit depth may vary along the slit. The slit width may extend to the full width of the septum along the entire septum, and the vascular access device may also include supports between the body and the septum. The supports may apply compressive force against the slit, for example, to encourage the slit towards a closed position. The slit may be molded.

The slit width may extend to the full width of the septum along the entire septum, and the slit width may form a crooked path between the first slit end and the second slit end. The septum may be formed of two substantially identical sides or halves. The outer diameter of the two sides or halves, when combined, may be greater than the inner diameter of the body where the septum is disposed.

A method of manufacturing a vascular access device may include providing a body and a septum. The body may have a first body end region and a second body end region and may define a passage extending through the body. The septum may include a first septum end region and a second septum end region, the septum may also include a slit extending from the first septum end region to the second septum end region, and the septum may be configured to resist tearing. The method may also include disposing at least a portion of the septum in the body to at least substantially seal the passage extending through the body. The slit of the septum may be adapted to provide selective passage through the septum and the body.

The septum of the method of manufacturing may have a longitudinal axis, the slit may have a slit width between a slit first end and a slit second end, the slit width may extend orthogonally to the longitudinal axis, and/or the septum may include a top disk. The method may also include reducing the thickness of the septum along a cross section of the septum that is parallel to the slit width. The method may also include relieving stress at the slit first end and the slit second end by providing at least one relief formation in communication with at least a portion of the slit within the top disk. Relief formations may include relief cuts, slots, holes, slits of deviating directions, and/or any other structure or lack thereof capable of relieving stress. The method may also include molding the slit within the septum, extending the slit width to the width of the septum, varying the depth of the slit within the septum, compressing the slit towards a closed position within the body, and/or deviating the path of the slit along the slit width between the slit first end and the slit second end.

A vascular access device may include a body means, a sealing means, and/or a means for discouraging slit tearing. The body means may be a means for selective coupling to a vascular system of a patient and to at least one additional medical device. The body means may have a passage extending through the body means. The sealing means may include a slit for selectively and at least substantially sealing the passage through the body.

These and other features and advantages of the present disclosure may be incorporated into vascular access devices and will become more fully apparent from the following description and appended claims, or may be learned by the practice and implementation of the present disclosure. As described above, the present disclosure does not require that all of the features described herein be incorporated into every embodiment nor is it required that certain features be used exclusive of other features. Vascular access devices within the scope of the present disclosure may include one or more combinations of the features described herein.

### BRIEF DESCRIPTION OF THE SEVERAL VIEWS OF THE DRAWINGS

In order that the above-recited and other features and advantages of the disclosure may be readily understood, a more particular description is provide below with reference to the appended drawings. These drawings depict only exemplary embodiments of vascular access devices according to the present disclosure and are not therefore to be considered to limit the scope of the disclosure.

Figure 1 is a perspective view of an extravascular system connected to the vascular system of a patient.

Figure 2 is a top view of a vascular access device.

Figure 3 is a perspective side view of a vascular access device.

Figure 4 is a cross section view of a vascular access device.

Figure 5 is a cross section view of a vascular access device with the cross section being 90 degrees offset from the cross section of Figure 4.

Figure 6 is a perspective view of a septum having a top disk of varying thickness.

Figure 7 is a top plan view of a septum having at least one relief cut.

Figure 8 is a side view of the septum of Figures 7 and 9.

Figure 9 is a cross section view of Figure 7 taken along lines 9-9.

Figure 10 is a top view of a top disk of a septum having at least one perpendicular relief cut.

Figure 11 is a cross section view of Figure 10 taken along lines 11-11.

Figure 12 is a top view of a top disk of a septum having at least one slot.

Figure 13 is a cross section view of Figure 12 taken along lines 13-13.

Figure 14 is a top view of a top disk of a septum having at least one hole therein.

Figure 15 is a cross section view of Figure 14 taken along lines 15-15.

Figure 16 is a perspective view of a septum having a slit with deviating ends.

Figure 17 is a top view of the top disk of the septum of Figure 16.

Figure 18 is a top view of a top disk of a septum having a slit extending along the entire width of the top disk.

Figure 19 is a side view of a septum having a uniform slit depth.

Figure 20 is a partial side view of a septum having a non-uniform slit depth.

Figure 21 is a top view of a septum encircled by a rigid material.

Figure 22 is a perspective view of a septum with a slit having a variable width and depth.

Figure 23 is a top perspective view of the septum of Figure 22.

Figure 24 is a cross section view of two halves of a septum.

Figure 25 is a cross section view of the two halves of the septum of Figure 24 combined with other structure to form a vascular access device.

Figure 26 is a cross section view of Figure 25 taken along lines 16-16.

Figure 27 is a top view of a top disk of a septum having a molded slit.

Figure 28 is a cross section view of a vascular access device having an injected foam material.

Figure 29 is a cross section view of a vascular access device having compression springs.

Figure 30 is a cross section view of a half of a septum having a slit that forms a crooked path.

Figure 31 is a top view of the half of Figure 30 combined with its corresponding other half.

Figure 32 is a top view of a top disk of a septum having two substantially identical halves.

Figure 33 is another embodiment of a top disk of a septum having two substantially identical halves.

### DETAILED DESCRIPTION OF THE INVENTION

It will be readily understood that the components of the present disclosure, as generally described and illustrated in the figures herein, could be arranged and designed in a wide variety of different configurations. Thus, the following more detailed description, as represented in the figures, is not intended to limit the scope of the disclosure, but is merely a representative of exemplary combinations of the components.

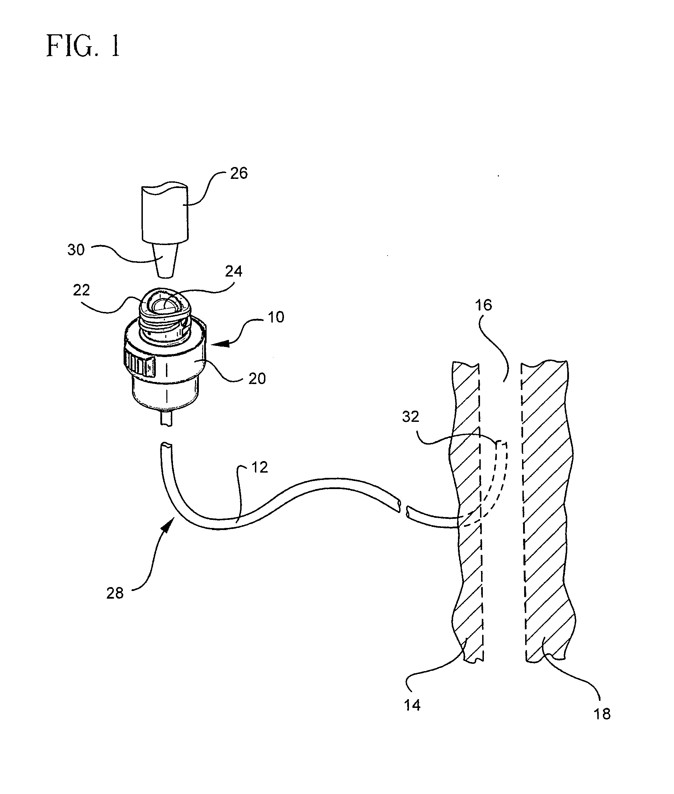

Referring now to Figure 1, a vascular access device 10 is used to introduce a substance via a catheter 12 across the skin 14 and into a blood vessel 16 of a patient 18. The vascular access device 10 includes a body 20 with a lumen and a septum 22 placed within the lumen. The vascular access device 10, including the body 20 and the septum 22, will be more thoroughly described with reference to the remaining figures where particular features are better illustrated. As shown in Figure 1, the septum 22 has a slit 24 through which a separate extravascular device 26, such as a syringe, may introduce a substance into the vascular access device 10. A syringe is one exemplary separate device 26. Other suitable extravascular devices may include additional vascular access devices, IV administration sets, or other common or yet to be developed medical devices.

The device 10 and all structures used in combination therewith may form a larger extravascular system 28. As part of operating the extravascular system 28, a tip 30 of the separate device 26 may be inserted into the vascular access device 10 through the slit 24 of the septum 22. The tip 30 penetrates the device 10 separating at least portions of the two opposing slit surfaces of the septum 22. The septum 22 and the slit 24 may be configured to seal, or at least substantially seal, around the tip 30 as it is inserted into the vascular access device 10. Accordingly, the surfaces near the slit ends may not be separated until the tip 30 is sufficiently inserted into vascular access device 10. The tip 30 serves to open the slit 24 to allow fluid to pass through the device 10, into the catheter 12, and out the end 32 of the catheter when the device is in use.

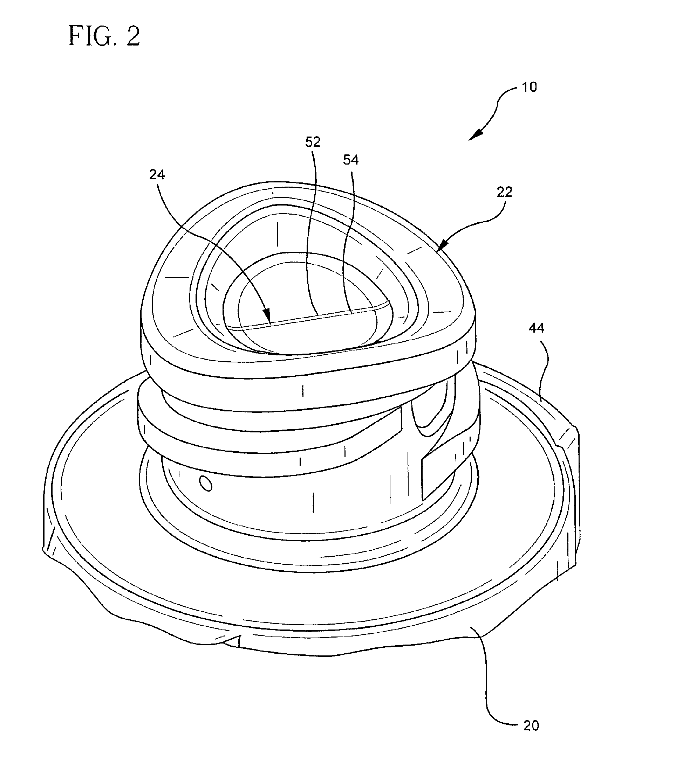

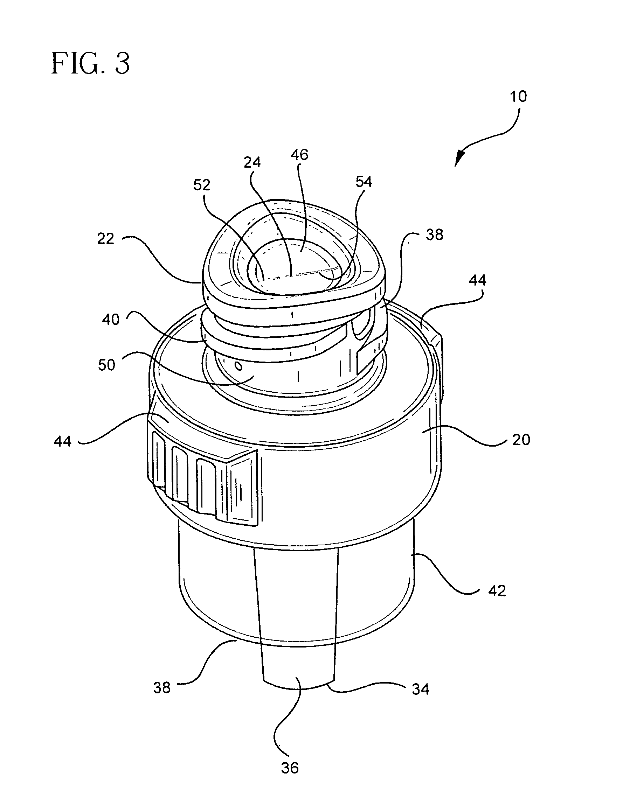

The features of an example of a vascular access device 10 are illustrated in Figures 2 and 3. As illustrated in these figures, the septum 22 includes a portion that extends beyond the body 20 but is otherwise disposed substantially within the body 20. The body 20 may include a cannula 34 for coupling with a catheter or other medical device. The cannula 34, along with other components of the body 20, may cooperate to form a lumen 36 through the body 20. The body 20 may also include connection regions 38, such as female Luer connector 40 or male Luer connector 42, to enable the vascular access device to be selectively coupled to other medical devices. Additional, the body 20 may include grips 44, which may be ridges or other structures on the surface of the body 20, to facilitate the manipulation of the vascular access device 10. The body 20 may include other features or structures common to vascular access devices.

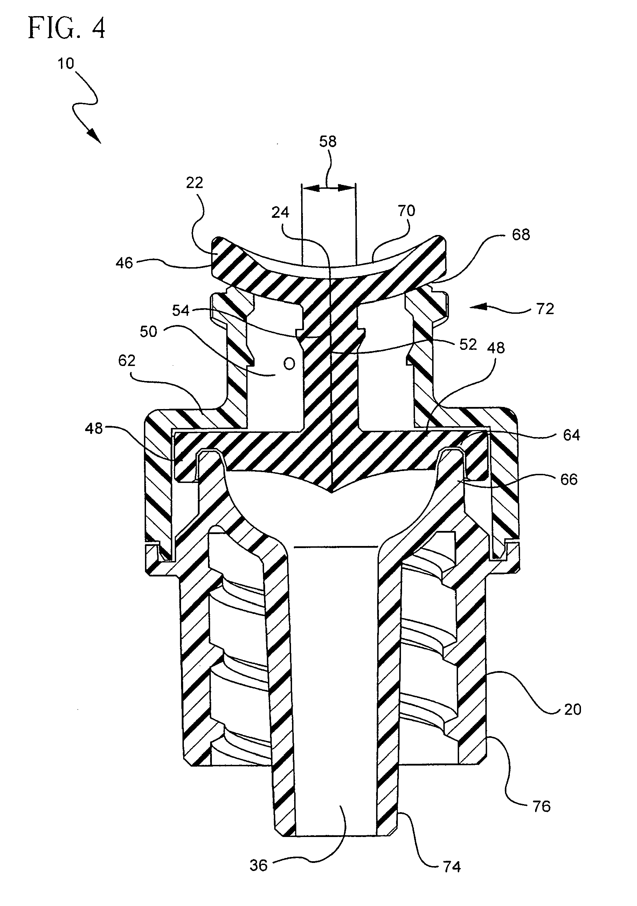

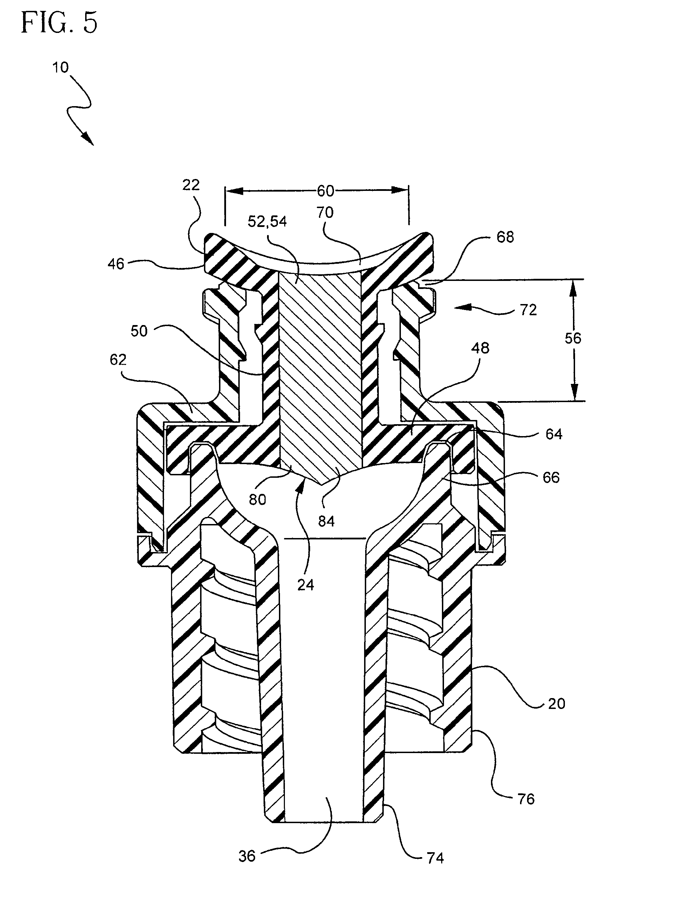

With continuing reference to Figures 2 and 3, the septum 22 is substantially disposed within the body 20 of the vascular access device 10. More specifically, the septum 22 includes a top disk 46, a bottom disk 48, and a throat region 50 extending between the top disk 46 and the bottom disk 48. The throat section 50 and bottom disk 46 are more clearly visible in the cross section view presented in Figures 4 and 5. As used herein, the top disk 46 may also be referred to as a saddle 46 and the bottom disk 48 may be referred to as an anchor 48. With more particular reference to Figure 2, the septum 22 is shown to include a slit 24 having opposing slit surfaces 52, 54. As described above, the opposing slit surfaces 52, 54 of the slit 24 are moved apart to open the slit when the tip 30 of a medical device is inserted into a vascular access device 10.

Referring now to Figures 4 and 5, cross sectional views of a vascular access device 10 are shown to better illustrate particular aspects of an exemplary septum 22. As illustrated, Figures 4 and 5 are cross sections of the same vascular access device with the cross sections being taken along orthogonal lines of cross section. Figure 4 illustrates a vascular access device 10 showing the throat region 50 spanning between the saddle 46 and the anchor disk 48. The throat region 50 may have any suitable length 56 between the saddle 46 and the anchor 48, which length 56 may vary to accommodate the configuration of the body 20. As one example, the length 56 may be selected to position the anchor disk 48 within the body 20 and the saddle 46 outside the body, as illustrated.

The throat region 50 also has a thickness 58, shown in Figure 4, and a width 60, shown in Figure 5. The width 60 and thickness 58 of the throat region 50 may be selected to meet the needs of the medical practitioner and the vascular access device 10 in which the septum 22 is being incorporated. The width 60 may be selected to provide sufficient room for a slit 24 sufficiently wide to accommodate the desired tips 30 of the cooperating medical devices 26. The thickness 58 of the throat region 50 may be selected to provide sufficient strength to the throat region while still providing sufficient elasticity and/or flexibility to allow the slit surfaces 52, 54 to separate as the tips 30 are inserted into the vascular access device 10.

The bottom disk 48, or anchor disk, may be configured to have a size, such as a diameter, that is selected to fit within the body 20 and to be retained in the body by a shoulder region 62. Additionally or alternatively, the bottom disk 48 may be anchored within the body 20 through other means, such as through adhesives or fasteners. As illustrated in Figures 4 and 5, the bottom disk 48 may include one or more grooves or slots 64 that may be adapted to cooperate with portions of the body 20 to further anchor the septum 22 in place. The bottom disk 48 and one or more portions of the body 20 may be configured to anchor the septum 22 rotationally within the body, longitudinally within the body, and/or laterally within the body. As one example, fingers 66 of the body 20 may be adapted to fit in the grooves 64 to prevent lateral movement and/or rotational movement of the septum 22. Additionally or alternatively, the fingers 66 may be sized to press the bottom disk 48 into the shoulder region 62 so that the top surface of the bottom disk is in contact with the body 20. As one example, the fingers 66 may cause the bottom disk 48 and the body 20 to form a seal. In addition to the features described, the bottom disk 48 may include additional features or elements customary for vascular access devices.

Figures 4 and 5 illustrate that the top disk 46 may be configured to be disposed outside of the body 20. As illustrated the bottom surface of the top disk 46 rests on the upper end 68 of the body 20. Figure 4 further illustrates that the top disk 46 may be configured to provide a well 70 or indentation. The well 70 may assist in guiding the tip 30 of the cooperating medical device 26 into the slit 24 of the vascular access device 10. As seen in Figures 4 and 5, the well 70, in some implementations, may cause the top disk 46 resemble a saddle. The well 70, when present, may be formed by thinning a portion of the top disk 46 and/or by applying upward pressure to the outside edge of the top disk 46. As one example, the septum 22 may be configured with a throat region 50 that is minimally shorter than the distance between the shoulder region 62 of the body 20 and the upper end 68 of the body. Accordingly, the septum 22 material of the throat region 50 and the top disk 46 may be slightly stretched by this difference causing the top disk to flex forming the well 70. The well 70 may be formed in other suitable manners.

As discussed above and as illustrated in Figure 4, the top disk 46 contacts the upper end 68 of the body 20. The interface between the top disk 46 and the upper end 68 of the body 20 may form an additional seal, which may be similar to the seal between the bottom disk 48 and the body 20. Additionally or alternatively, an adhesive may be used to bond the top disk 46 to the upper end 68 of the body. Moreover, structural features, such as grooves, may be incorporated into the bottom surface of the top disk 46 to cooperate with the body 20 to form a seal. The seals formed by the top disk 46 and/or the bottom disk 48 and the body 20 are adapted to seal, or at least substantially seal the lumen 36 through the body 20. Moreover, when the slit surfaces 52, 54 are together (i.e., not separated by a tip 30 and not otherwise separated by tears, cracks, or other modifications to the septum 22), the septum 22 seals, or at least substantially seals the passage through the lumen of the body 20.

For purposes of description, the upper end 68 of the body 20 and the portions adjacent thereto may be referred to as a first body end region 72 whereas the lower end 74 of the body 20 and the portions adjacent thereto may be referred to as the second body end region 76. The use of the terms first and second to denominate the end regions, or other elements described herein, is not meant to imply any order between the two end regions but merely to distinguish between the two. While the terms top and bottom are also used herein to designate and distinguish features, components, or parts of the vascular access device, it should be understood that the orientation of the vascular access device may change during use of the device; accordingly, the terms top and bottom are not intended to be limiting with respect to orientation during use of the device but are referencing relative locations in the figure being discussed.

The body 20 and the septum 22 may be constructed of a variety of suitable materials. Commonly, the body 20 of the vascular access device 10 will be made of a plastic, and preferably a plastic material that facilitates molding the body. As illustrated in Figures 4 and 5, the body 20 is formed from two pieces that are molded or adhered together to form the body once the septum 22 is in place. Other methods and materials may be used for manufacturing the body 20, some of which may be currently practiced and some of which may be developed in the future.

Similarly, the septum 22 may be made of a variety of suitable materials and through a variety of suitable manufacturing methods. For example, the septum 22 may be formed from liquid silicone rubber through suitable molding procedures, such as insert molding, injection molding, other molding techniques, or a combination of molding techniques. The septum 22, the body 20, and/or other features or elements of a vascular access device 10 may be modified or configured as needed or desired to resist tearing of the slit 24. Such configurations or modifications will be described with reference to the following figures.

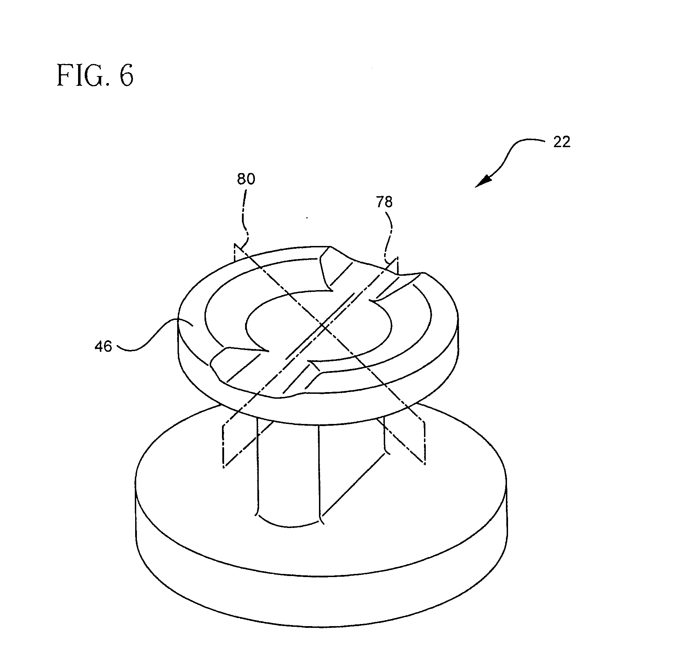

Referring now to Figure 6, a septum 22 includes a top disk 46 and a slit 24 passing through at least the top disk 46 of the septum 22. The top disk 46 of the septum 22 is configured to resist tearing near the slit 24. A first cross section taken along the line 78 is parallel to the slit 24. Any number of other cross sections that are not parallel to the slit 24 may be taken. For example, the cross section taken along line 80 that intersects with the slit 24 may be taken. The cross section taken along line 78 indicates a relatively thin portion of the top disk 46. By contrast, the cross section taken along line 80 yields a thicker portion of the top disk 46. The thinner portions of the top disk 46 exist near the ends of the slit 24. The regions of material near the ends of the slit 24 are thinner than the remaining surrounding regions of material of the top disk 46 in order to provide a more resilient and flexible region in which the slit 24 may expand into open position during access by a separate vascular access device 26. By providing a more resilient and flexible region of material, the material of the top disk 46 will be less likely to tear, and be less brittle, than the thicker regions of material around the edges of the top disk 46, such as those measured by the cross section taken along line 80. Thus, the embodiment described with reference to Figure 6 provides a septum 22 having a top disk 46 with a reduced thickness of the top disk 46 at the ends of the slit 24 in order to reduce the stress placed upon the material within those regions.

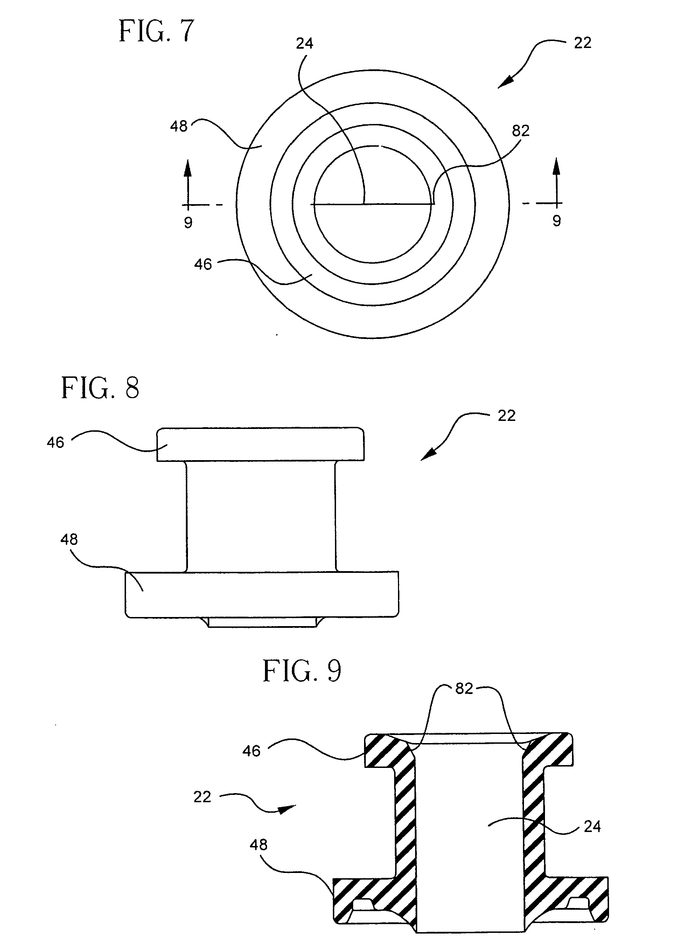

Referring now to Figure 7, a septum 22 includes a top disk 46, a bottom disk 48, and a slit 24 within the top disk 46 and having at least one (1) relief cut 82. The at least one relief cut 82 is in line, or parallel with, the path of the slit 24. The relief cuts 82 exist at the ends of the slit 24 in order to provide additional relief to the material surrounding the ends of the slit 24 when the slit 24 is opened during insertion of a separate vascular access device 26. A cross section taken along lines 9-9 of Figure 7 is shown in Figure 9.

Referring now to Figure 9, the septum 22 described with reference to Figure 7 is shown in cross section view, revealing the top disk 46, the bottom disk 48, a planar view of the slit 24, and the relief cuts 82 in communication with the slit 24. In this embodiment, the relief cuts 82 are cut at a V-shaped angle on the edges of the slit 24 in order to provide a narrowing access into the slit 24 through which a separate device 26 may be inserted. The graduated, or narrowing entry into the slit 24 by means of the slit reliefs or relief cuts 82 will provide less initial shock and stress upon the material surrounding the edges of the slit 24 within the top disk 46 where the separate access device 26 first comes into contact with the septum 22. By providing less initial stress and shock, the relief cuts 82 reduce the likelihood that the slit 24 will tear during access. Figure 8 is a side view of the septum 22 described with reference to Figures 7 and 9.

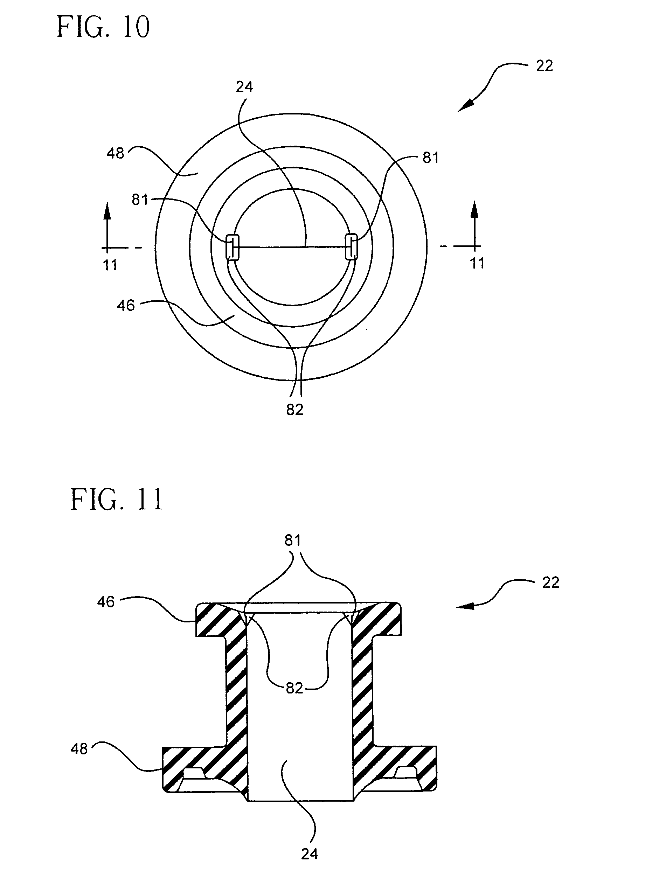

Referring now to Figure 10, a septum 22 may include a top disk 46 and a slit 24 in communication with at least one relief cut 82. The relief cuts 82 are perpendicular to the direction of the primary width of the slit 24. The relief cuts 82 may be molded within the same or a different material 84 as the material of the top disk 46. By molding the relief cuts 82 separately in the material 84 from the remaining portion of the top disk 46, the edges of the relief cuts 82 will be less likely to tear or progress beyond the molded border between the separate material 84 and the remaining material of the top disk 46. This process of molding slit relief cuts 82 in addition or alternate to molding of the slit 24 itself, may in certain circumstances yield less tearing of the slit 24 within the septum 22. The top view of the septum 22 shown in Figure 10 is shown as a cross section view taken along lines 11-11 of Figure 10 in Figure 11.

Referring now to Figure 11, the septum 22 described with reference to Figure 10 is shown in cross section view taken along lines 11-11 of Figure 10. The septum 22 includes a top disk 46, a slit 24, and relief cuts 82 formed in communication with the slit 24. Similar to the embodiment described with reference to Figures 7 through 9, the relief cuts 82 provide a narrowing or graduated tapering as the cuts 82 progress deeper into the top disk 46. This narrowing or graduated tapering provides a gentle slope into which a separate vascular access device 26 may be inserted in a manner that decreases the likelihood that the slit 24 will tear.

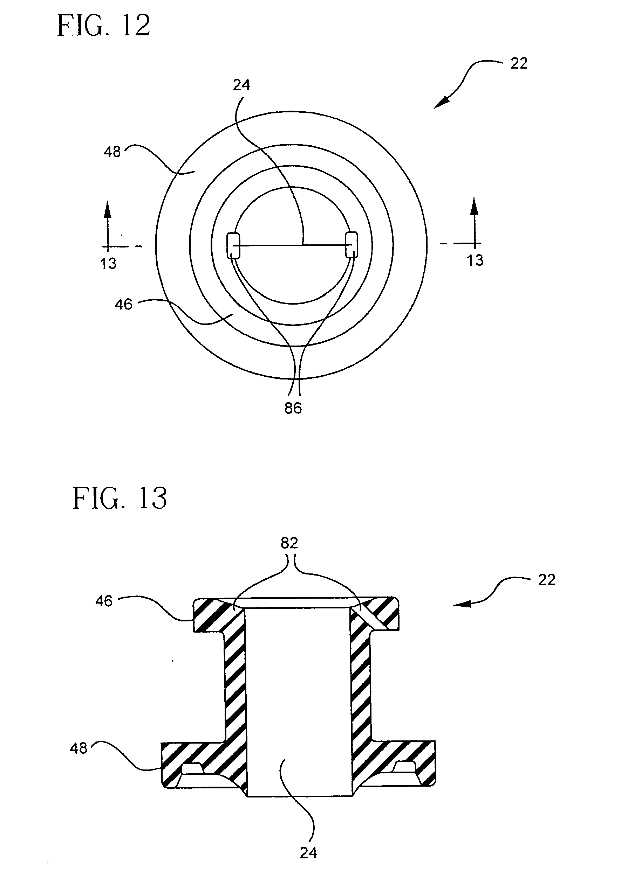

Referring now to Figure 12, a septum 22 may include a top disk 46, a slit 24, and at least one relief cut 82 that is a slot. The slots or relief cuts 82 may extend through the full depth of the top disk 46 as shown in the cross section of Figure 12 taken along lines 13-13.

Referring now to Figure 13, a cross section view of Figure 12 taken along lines 13-13 is shown. The cross section view reveals the septum 22 having the top disk 46, the slit 24, and the slots or relief cuts 82 extending through the top disk 46. The slots, which may also exist as dimples or other similar structures or lack thereof, may extend to any depth within the septum 46 in order to provide a structure which redirects the stress that is exerted upon the material of the top disk 46 surrounding the slit 24 when a separate vascular access device 26 is inserted into the septum 22. The relief cuts 82 widen in a direction from the top surface of the top disk 46 to the bottom surface of the top disk 46. The widening of the relief cuts 82 permits the stress placed upon the material surrounding the relief cuts 82 and the slit 24 to be directed away from the slit 24 as the tip 30 of a separate device 26 is advanced into the slit 24. Since configurations of the relief cuts 82 such as that shown in Figure 13 may lead to leakage of fluids from the slit 24 into the relief cuts 82 and to the bottom surface of the top disk 46, other orientations or configurations of the relief cuts 82 are possible. For example, the relief cuts 82 may terminate prior to breaching the bottom surface of the top disk 46, so that no leaking of fluids may occur through the relief cuts 82.

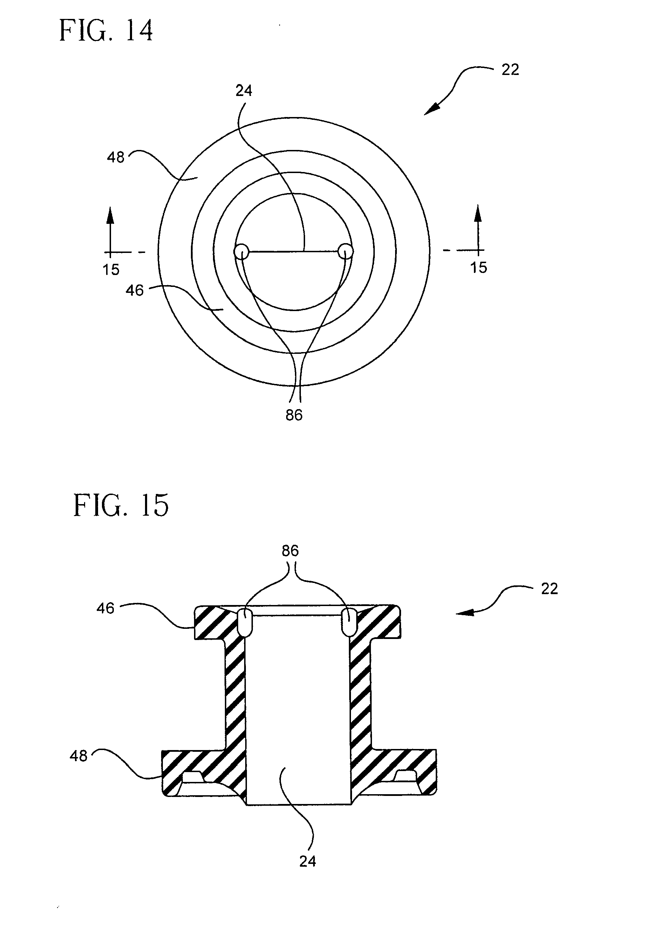

Referring now to Figure 14, a septum 22 may include a top disk 46, a slit 24 formed within at least the top disk 46, and at least one hole 86 at which the ends of the slit 24 may terminate. Similar to relief cuts 82 and other features described herein, the at least one hole 86 provides an area of relief from stress that is exerted upon the material within the region of the ends of the slit 24. By providing this relief, and by providing a structure or lack of structure which is likely to redirect the force that a simple straight slit end would create, the slit 24 will be less likely to propagate tearing at its ends.

Referring now to Figure 15, the septum 22 described with reference to Figure 14 is shown in cross section view, taken along lines 15-15 of Figure 14. The septum 22 reveals a bottom disk 48, a top disk 46, a slit 24, and holes 86 located within the top disk 46 at the ends of the slit 24. The holes 86, similar to the relief cuts 82 and other features described herein, may provide a narrowing or graduated tapering as the holes extend down through the material of the septum 22. Thus an abrupt stress that would normally be likely to cause tearing of the material of the septum 22 is less likely to occur upon insertion and advancement of the tip 30 of a separate vascular access device 26 through the slit 24. The slit 24 and the holes 86 may be manufactured using molding, cutting, drilling, boring, or other techniques in any order and/or combination. For example, the holes 86 may be manufactured prior to manufacturing of the slit 24, and visa versa.

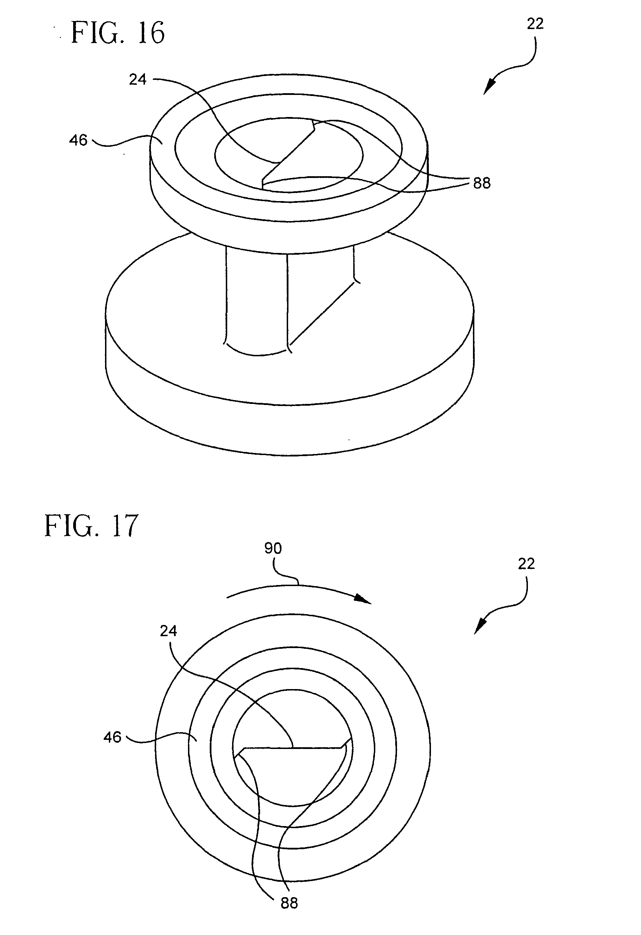

Referring now to Figure 16, a septum 22 may include a top disk 46, a slit 24 within the top disk 46, and at least one slit end 88. For example, the at least one slit end 88 travels in a direction inconsistent with the straight line of the majority of the slit 24. The at least one slit end 88 travels in a direction that is opposite the direction that a separate vascular access device 26 would rotate when accessing the slit 24 of the septum 22.

Referring now to Figure 17, a top view of the top disk 46 of the septum 22 is shown. The top view of the top disk 46 reveals the slit 24 with the slit ends 88 traveling in a direction that is opposite the rotational direction 90 that a separate vascular access device 26 would travel when initially accessing the slit 24 of the septum 22. Thus, where the vascular access device rotation 90 is clockwise, the slit ends 88 will deviate from the straight line of the slit 24 in a direction that is counterclockwise. By providing slit ends 88 that travel in a direction that is opposite the direction of the rotation 90, the slit ends 88 will serve to resist the propagation of a tear in the slit 24 which would normally occur under the rotational influence of a separate vascular access device 26.

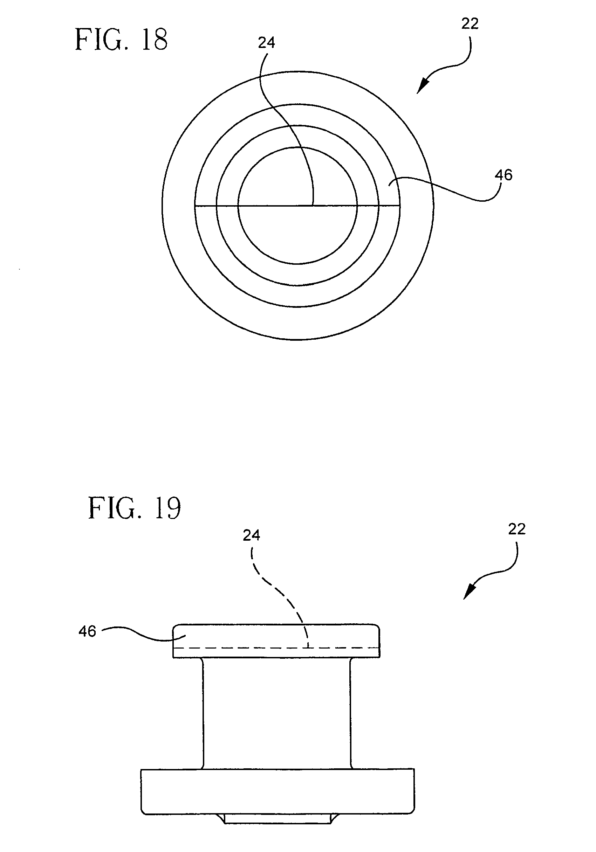

Referring now to Figure 18, a top view of a top disk 46 of a septum 22 shows a slit 24 that extends to the full-width of the top disk 46 of the septum 22. The slit 24 may be cut, molded, or otherwise formed during manufacturing. The slit 24 may extend to any depth within the top disk 46 and/or the remainder of the septum 22.

Referring now to Figure 19, a side view of the septum 22 described with reference to Figure 18 is shown illustrating the top disk 46, the slit 24, and a bottom disk 48. As shown in Figure 19, the slit 24 extends to a depth that is just over half of the full depth of the top disk 46 and is substantially constant across the entire width of the top disk 46. However, as mentioned previously, the depth of the slit 24 may vary.

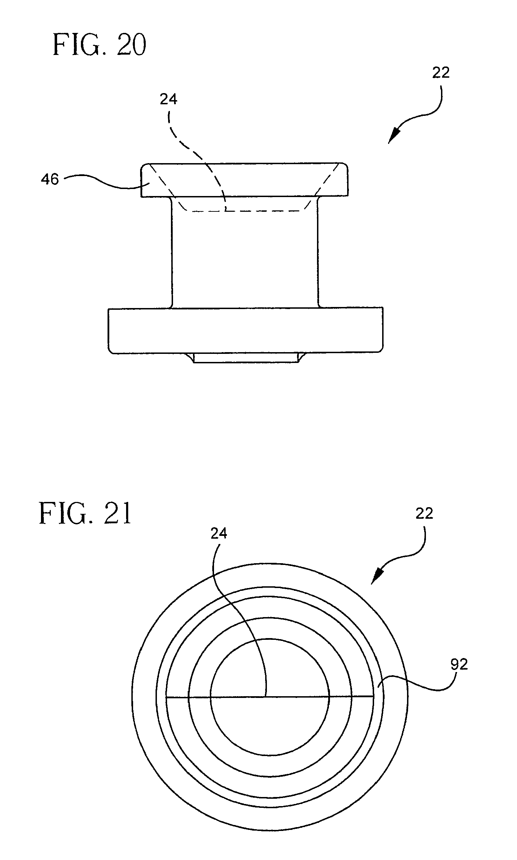

For example, as shown in Figure 20, slit 24 depth may vary along the width of the top disk 46 to provide a deeper slit 24 near the center portion of the top disk 46 and a shallower depth of the slit 24 near the edges of the top disk 46. This graduated and narrowing slit 24 depth may facilitate the graceful entry of a tip 30 of a separate vascular access device 26 that is accessing the septum 22, yielding less likelihood that the material near the slit 24 will tear. Various other slit 24 depths and shapes are possible.

Referring now to Figure 21, the septum 22 described with reference to any of the figures described herein, such as Figures 18 through 20, may further include a relatively rigid structure 92 surrounding the septum 22 in order to apply a degree of compression, stability, and/or structure to the operation of the slit 24 within the septum 22. The rigid structure 92 may be the body 20 of the vascular access device 10 or may be any other structure or material attached to the body 20. The material of the rigid structure 92 may include an injected plastic ring, such as polystyrene and/or polycarbonate. During manufacturing, the septum 22 may first be prepared, after which the septum 22 may be forced into compression and the rigid structure 92 may be injected surrounding the compressed septum 22. After the rigid structure 92 has cured, the compression of the septum 22 may be released against the inner surface of the rigid structure 92.

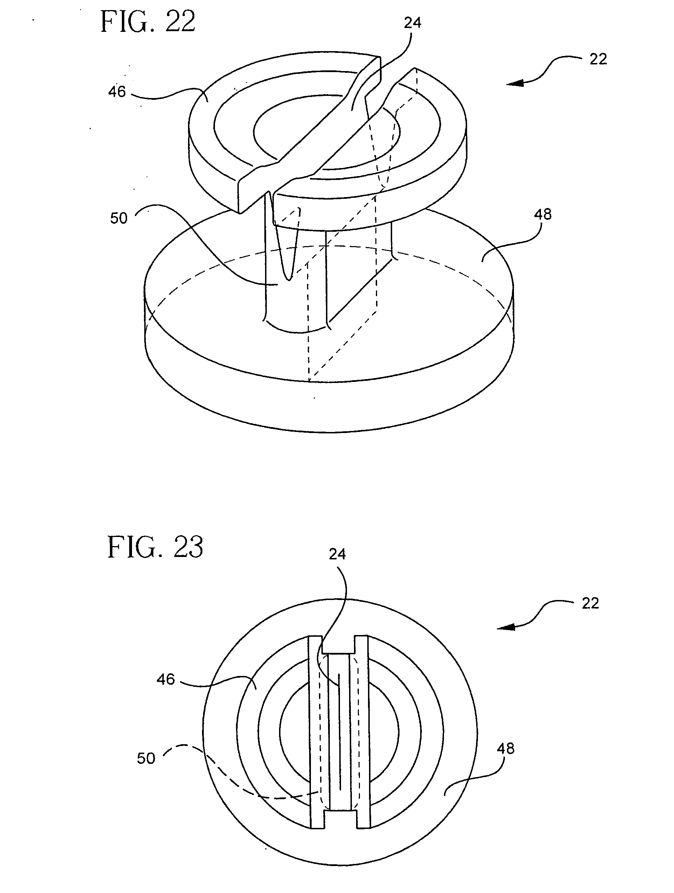

Referring now to Figure 22, a septum 22 includes a bottom disk 48, a top disk 46, and a slit 24 extending through the septum 22. The slit 24 extends across the full width of the top disk 46 and across the full width of a majority of a throat region 50 between the top disk 46 and the bottom disk 48. The slit 24 then continues through the remainder of the throat region 50 and through the bottom disk 48 with a narrower width.

Referring now to Figure 23, the septum 22 described with reference to Figure 22 is shown in top perspective view. The top perspective view of the septum 22 illustrates the top disk 46 split by the slit 24, and the narrow width of the slit 24 extending through the remaining portion of the throat region 50 and the bottom disk 48. Thus, the embodiment described with reference to Figures 22 and 23 reveals a slit 24 which extends through the full depth of the septum 22 along a longitudinal axis.

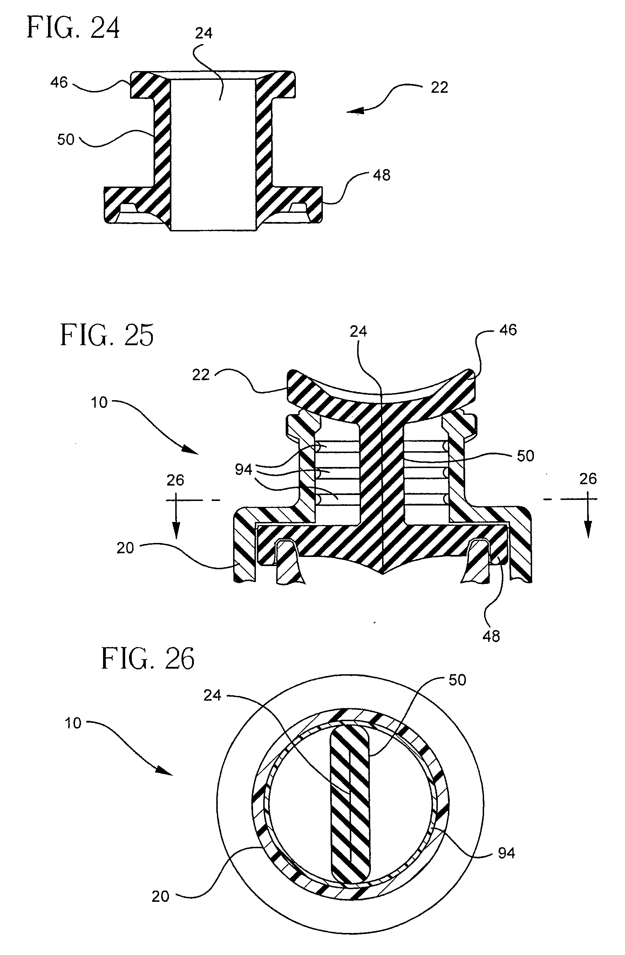

The slit 24 depth varies along the slit, such that only a portion of the slit 24 extends through the entire depth of the septum 22. The septum 22 described with reference to Figures 23 and 24 and other septa described herein may require structure in addition to the body 20 of the device 10 in order to provide adequate support for proper operation of the slit 24 within the septa. For example, as previously described, a rigid supporting structure 92 may be used as was discussed with reference to Figure 22. Various other supporting structures may be provided and will be described with reference to the following figures.

Referring now to Figure 24, a septum 22 may be fully split along a slit 24 providing two separate halves. Each half may include a half or section of a top disk 46, a throat region 50, and a bottom disk 48. Such a septum 22 will likely require supporting structure in addition to the body 20 of a vascular access device 10 in order to function properly.

Referring now to Figure 25, the septum 22 described with reference to Figure 24 is shown housed within the body 20 of a vascular access device 10. The two halves of the septum 22 are secured by additional supporting structures or supports 94 placed between the body 20 and the septum 22. The supports 94 apply compressive force against the slit 24, by providing force against the material of the septum 22 adjacent the slit 24. Such compressive force ensures that the slit 24 remains closed when the device 10 is not accessed by any separate access device 26.

Referring now to Figure 26, a cross section of the device 10 described with reference to Figure 25 is taken along lines 26-26 of Figure 26. The cross section view reveals the throat region 50 of the septum 22 and the slit 24 extending across the width of the throat region 50. The throat region 50 communicates with the inner surface of the body 20 of the device 10 at the ends of the throat region 50. Supports 94 frame the inner surface of the body 20 and compress against the ends of the throat region 50 to ensure stability of the septum 22. Various other supports 94 are possible and may be applied as needed or preferred with any of the embodiments described herein. Examples of such supports 94 will be described with reference to the following figures.

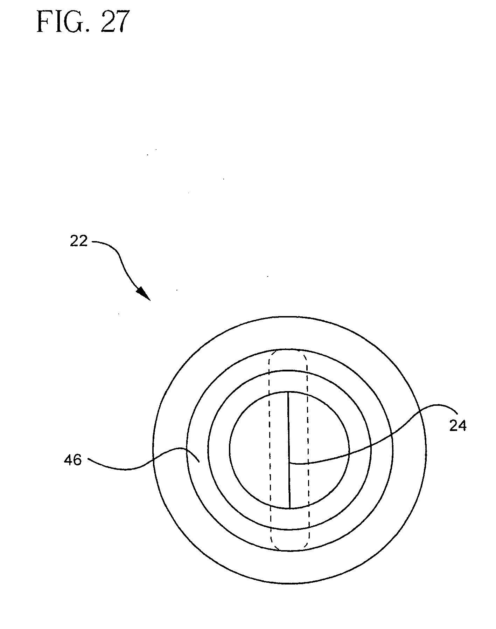

Referring now to Figure 27, a top view of a top disk 46 of a septum 22 may include a slit 24 that is molded, for example using a thin core pin, in an open position. Without any other force applied against the slit 24, the slit 24 will leak fluid during its operation within a vascular access device 10. Thus, additional support may be needed or preferred.

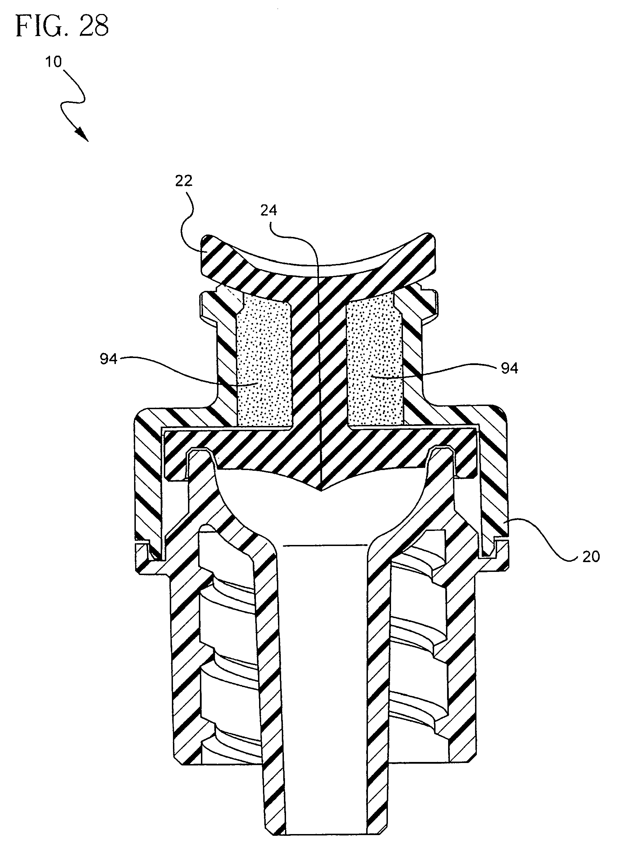

Referring now to Figure 28, a vascular access device 10 may include a body 20, a septum 22 having a slit 24, and at least one support 94 between the body 20 and the septum 22. The at least one support 94 applies compressive force against the slit 24 to encourage the slit 24 towards a closed position. The at least one support 94 may include a foam material that is injected, inserted, or otherwise placed between the septum 22 and the body 20.

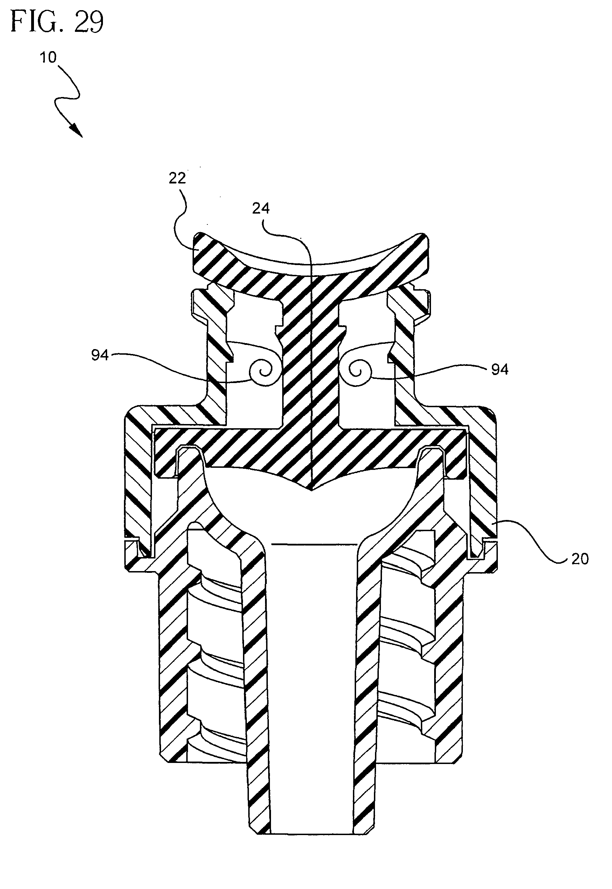

Referring now to Figure 29, a vascular access device 10 may include a body 20, a septum 22 having a slit 24 at least partially housed within the body 20, and at least one support 94. The at least one support 94 may reside between the body 20 and the septum 22. The at least one support 94 may be a spring or other support member capable of applying compressive force against the slit 24 in order to encourage the slit 24 towards a closed position.

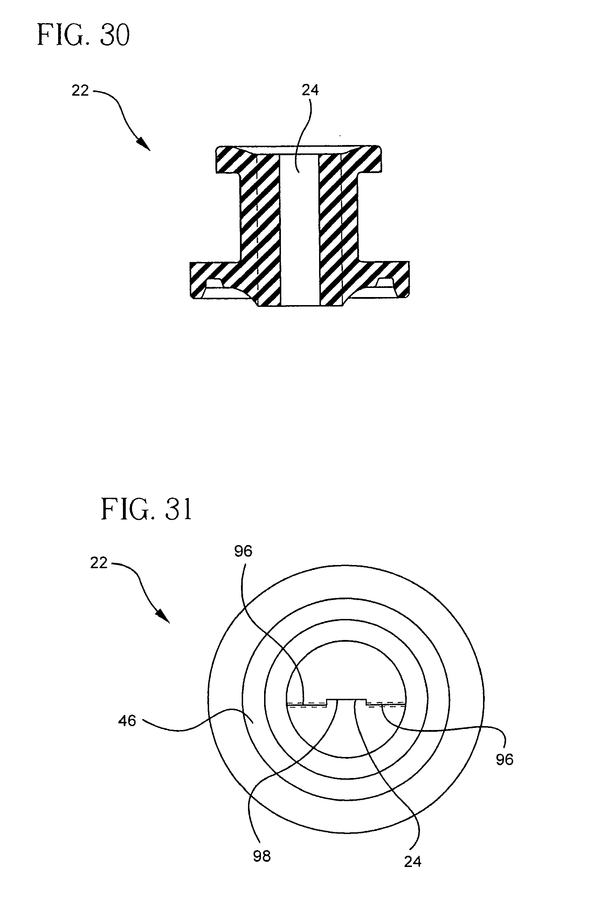

Referring now to Figure 30, a septum 22 may include a slit 24 which forms a crooked path along the entire width of the slit 24. Referring now to Figure 31, a top view of the septum 22 described with reference to Figure 30 is shown. The top view reveals the slit 24 extending along the entire width of a top disk 46 of the septum 22 in a crooked path. Since the slit 24 extends along the entire width of the top disk 46, the septum 22 is formed of two sides, portions, and/or halves. The outer diameter of the two sides, when combined, may be greater than the inner diameter of the body 20 where the septum 22 may be disposed when combined with the body 20 to form a vascular access device 10. For example, the combined width of the two halves or two sides may be 0,0508 mm (0.002 inches) greater than the inner width of the body 20 of a vascular access device 10.

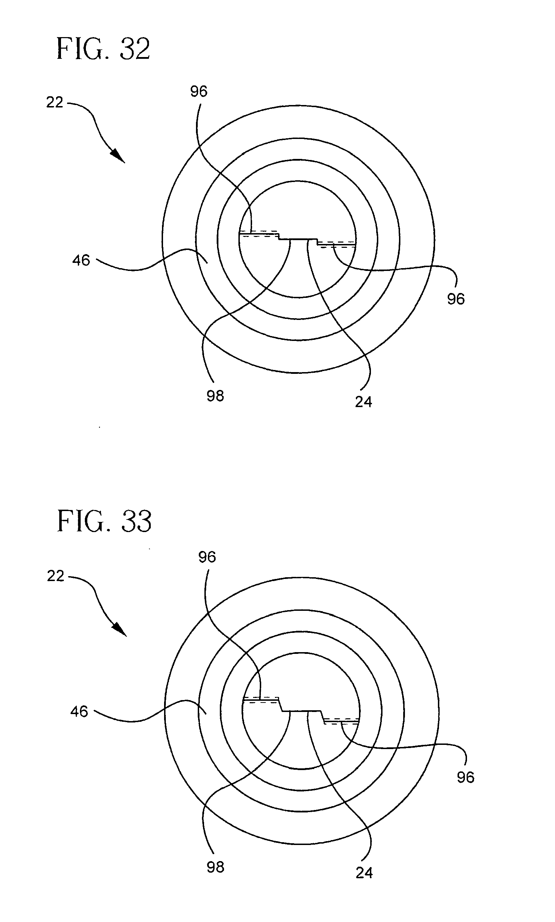

Various portions, preferably the outer portions, of the slit 24 may be bonded using adhesive or another substance, technique, and/or structure, to provide added sealing and structural stability to the slit 24. For example, the bond areas 96 of the slit 24 have been secured using adhesive. As the tip 30 of a separate vascular access device 26 is inserted through the center of the slit 24, the unbonded portion 98 will open with minimal force, until the unbonded portion 98 opens to the extent of exerting force upon the neighboring bonded portions 96. If the tip 30 continues to advance and open the unbonded portion 98, eventually the force exerted upon the septum 22 will become great enough to begin to open the bonded portions 96 of the slit 24. Such bonded portions 96 will open only to the extent that the force is great enough to open such bonded portions 96. Further, such bonded portions will open in a uniform and controlled manner based on the bond strength of such sections and will ensure that no material of the septum 22 is torn during insertion of the tip 30. Embodiments alternate or additional to the embodiment described with reference to Figures 30 and 31 are possible and examples of such embodiments will be described with reference to Figures 32 and 33.

Referring now to Figure 32, a septum 22 may include a top disk 46 including two separate and substantially identical sides. The two substantially identical sides are separated along the full width of the top disk 46 by a slit 24. The slit 24 may include bonded and unbonded portions 96 and 98 respectively. The slit 24 may form a crooked path between its ends as it traverses the width of the top disk 46. The crooked path of the slit 24 may be formed by a zigzag of substantially right angles as shown in Figure 32. Such angles may be increased, decreased, rounded, or otherwise adjusted depending on the needed or desired use of the septum 22. For example, as shown in Figure 33, the angles of the crooked path of the slit 24 may be rounded as they change the course of the path of the slit 24 from a bonded portion 96 to a central unbonded portion 98 and back to a bonded portion 96.

Any of the features or elements described in any of the embodiments herein may be combined with any other feature or element described herein to achieve the purposes of the invention as claimed. It is believed that the disclosure set forth above encompasses multiple distinct inventions with independent utility. While each of these inventions has been disclosed in its preferred form, the specific embodiments thereof as disclosed and illustrated herein are not to be considered in a limiting sense as numerous variations are possible. The subject matter of the inventions includes all novel and non-obvious combinations and subcombinations of the various elements, features, functions and/or properties disclosed herein. Where the disclosure, the presently filed claims, or subsequently filed claims recite "a" or "a first" element or the equivalent thereof, it should be within the scope of the present inventions that such disclosure or claims may be understood to include incorporation of one or more such elements, neither requiring nor excluding two or more such elements.

Applicants submit claims herewith and reserve the right to submit claims directed to certain combinations and subcombinations that are directed to one of the disclosed inventions and are believed to be novel and non-obvious. Inventions embodied in other combinations and subcombinations of features, functions, elements and/or properties may be claimed through amendment of those claims or presentation of new claims in that or a related application. Such amended or new claims, whether they are directed to a different invention or directed to the same invention, whether different, broader, narrower or equal in scope to the original claims, are also regarded as included within the subject matter of the inventions of the present disclosure.
The method of claim 11 further comprises relieving stress at the slit first end and the slit second end by providing at least one relief formation in communication with at least a portion of the slit within the top disk.
Preferably, the method further comprising extending the slit width to the width of the septum.
Preferably, the method further comprising varying the depth of the slit within the septum.
The method further comprising compressing the slit towards a closed position within the body.
According to a further aspect of the invention, a vascular access device comprises:
a body means for selective coupling to a vascular system of a patient and to at least one additional medical device and having a passage extending through the body means;
a sealing means including a slit for selectively and at least substantially sealing the passage through the body; and
a means for discouraging slit tearing.

## Claims

1. A vascular access device, comprising:
a body (20) defining a lumen (36) extending through the body (20); and
a septum (22) at least partially disposed in the body (20) to at least substantially seal the lumen (36) extending through the body (20);
wherein the septum (22) has a longitudinal axis;
wherein the septum (22) has a slit (24) adapted to provide selective passage through the septum (22);
wherein the slit (24) has a slit width between a slit first end and a slit second end; and
wherein the slit width extends orthogonally to the longitudinal axis;
**characterized in that** the septum (22) includes a top disu (46) and
at least a portion of the slit within the top disk has at least one relief cut, so that the septum comprises a structure which resists tearing near the slit (24).

2. The vascular access device of claim 1, wherein the top disk (46) includes a first cross section and a second cross section, wherein the first cross section is parallel to the slit and is thinner than the second cross section.

3. The vascular access device of claim 1, wherein the at least one relief cut is in line with the slit width and wherein preferably at least a portion of the at least one relief cut is perpendicular to the slit width.

4. The vascular access device of claim 1, wherein the first slit end and the second slit end each terminate at a hole (86) within the top disk (46).

5. The vascular access device of claim 1, wherein the first slit end (88) and the second slit end (88) each travel in a direction opposite the direction that a separate vascular access device would rotate when accessing the septum (22).

6. The vascular access device of claim 1, wherein the slit width extends to the full width of the septum (22) at the top disk (46).

7. The vascular access device of claim 1, further comprising supports between the body (20) and the septum (22), wherein the supports apply compressive force against the slit (24) to encourage the slit (24) towards a closed position.

8. The vascular access device of claim 6, wherein the slit width extends to the full width of the septum (22) along the entire septum (22), and wherein the slit width forms a crooked path between the first slit end and the second slit end.

9. The vascular access device of claim 8, wherein the septum (22) is formed of two substantially identical sides.

10. The vascular access device of claim 8, wherein the outer diameter of the two sides, when combined, is greater than the inner diameter of the body (20) where the septum (22) is disposed.

11. A method of manufacturing a vascular access device, comprising:
providing a body (20) having a first body end region and a second body end region and defining a passage extending through both body end regions;
providing a septum (22), wherein the septum includes a first septum end region and a second septum end region, wherein the septum further includes a slit (24) extending from the first septum end region to the second septum end region, disposing at least a portion of the septum (22) in the body (20) to at least substantially seal the passage extending through the body (20), wherein the slit (24) of the septum (22) is adapted to provide selective passage through the septum and the body (20); and
wherein the septum (22) has a longitudinal axis; wherein the slit (24) has a slit width between a slit first end and a slit second end; wherein the slit width extends orthogonally to the longitudinal axis; and wherein the septum (22) includes a top disk,
**characterized by**
relieving stress at the slit first end and the slit second end by providing at least one relief formation in communication with at least a portion of the slit (24) within the top disk so that the septum (22) includes a structure which resists tearing.

12. The method of claim 11, further comprising extending the slit width to the width of the septum (22).

13. The method of claim 11, further comprising varying the depth of the slit (24) within the septum (22).

14. The method of claim 11, further comprising compressing the slit (24) towards a closed position within the body (20).

## Patentansprüche

1. Gefäßzugangsvorrichtung mit:
einem Körper (20), der ein sich durch den Körper (20) erstreckendes Lumen (36) begrenzt; und
einem Septum (22), das zumindest teilweise in dem Körper (20) angeordnet ist, um das sich durch den Körper (20) ersteckende Lumen (36) zumindest teilweise abzudichten;
wobei das Septum (22) eine Längsachse aufweist;
wobei das Septum (22) einen Schlitz (24) aufweist, der geeignet ist, einen selektiven Durchtritt durch das Septum (22) zu ermöglichen;
wobei der Schlitz (24) eine Schlitzweite zwischen einem ersten Schlitzende und einem zweiten Schlitzende aufweist; und
wobei sich die Schlitzweite orthogonal zur Längsachse erstreckt;
**dadurch gekennzeichnet, dass** das Septum (22) eine obere Scheibe (46) aufweist und zumindest ein Teil des Schlitzes in der oberen Scheibe zumindest einen Entlastungsschnitt aufweist, so dass das Septum eine Struktur aufweist, welche einem Reißen nahe dem Schlitz (24) widersteht.

2. Gefäßzugangsvorrichtung nach Anspruch 1, bei welcher die obere Scheibe (46) einen ersten Querschnitt und einen zweiten Querschnitt aufweist, wobei der erste Querschnitt parallel zu dem Schlitz verläuft und dünner als der zweite Querschnitt ist.

3. Gefäßzugangsvorrichtung nach Anspruch 1, bei welcher der mindestens eine Entlastungsschnitt mit der Schlitzbreite ausgerichtet ist, und wobei vorzugsweise zumindest ein Teil des mindestens einen Entlastungsschnitts senkrecht zur Schlitzbreite verläuft.

4. Gefäßzugangsvorrichtung nach Anspruch 1, bei welcher das erste Schlitzende und das zweite Schlitzende jeweils an einem Loch (86) in der oberen Scheibe (46) enden.

5. Gefäßzugangsvorrichtung nach Anspruch 1, bei welcher das erste Schlitzende (88) und das zweite Schlitzende (88) sich jeweils in eine Richtung erstrecken, die zu der Richtung entgegengesetzt ist, in welcher sich eine separate Gefäßzugangsvorrichtung beim Zugang zum Septum (22) drehen würde.

6. Gefäßzugangsvorrichtung nach Anspruch 1, bei welcher sich die Schlitzbreite übe die gesamte Breite des Septums (22) an der oberen Scheibe (46) erstreckt.

7. Gefäßzugangsvorrichtung nach Anspruch 1, ferner mit Stützen zwischen dem Körper (20) und dem Septum (22), wobei die Stützen auf den Schlitz (24) eine Druckkraft aufbringen, um den Schlitz (24) in eine geschlossene Stellung vorzuspannen.

8. Gefäßzugangsvorrichtung nach Anspruch 6, bei welcher sich die Schlitzbreite über die gesamte Breite des Septums (22) entlang des gesamten Septums (22) erstreckt, und wobei die Schlitzbreite einen unregelmäßigen Verlauf zwischen dem ersten Schlitzende und dem zweiten Schlitzende aufweist.

9. Gefäßzugangsvorrichtung nach Anspruch 8, bei welcher das Septum (22) durch zwei im Wesentlichen identische Seiten gebildet ist.

10. Gefäßzugangsvorrichtung nach Anspruch 8, bei welcher der Auendurchmesser der beiden Seiten kombiniert größer als der Innendurchmesser des Körpers (20) bei angebrachtem Septum (22) ist.

11. Verfahren zur Herstellung einer Gefäßzugangsvorrichtung mit den folgenden Schritten:
Vorsehen eines Körpers (20, der einen ersten Körperendbereich und einen zweiten Körperendbereich aufweist und einen sich durch beide Körperendbereiche hindurch erstreckenden Durchgang begrenzt;
Vorsehen eines Septums (22), wobei das Septum einen ersten Septumendbereich und einen zweiten Septumendbereich aufweist, wobei das Septum ferner einen Schlitz (24) aufweist, der sich vom ersten Septumendbereich zum zweiten Septumendbereich erstreckt,
Anordnen zumindest eines Teils des Septums (22) in dem Körper (20), um den sich durch den Körper (20) erstreckenden Durchgang zumindest im Wesentlichen abzudichten, wobei der Schlitz (24) des Septums (22) geeignet ist, einen selektiven Durchtritt durch das Septum und den Körper (20) zu ermöglichen, und
wobei das Septum (22) eine Längsachse aufweist; wobei der Schlitz (24) eine Schlitzbreite zwischen einem ersten Schlitzende und einem zweiten Schlitzende aufweist; wobei sich die Schlitzbreite orthogonal zur Längsachse erstreckt; und wobei das Septum (22) eine obere Scheibe aufweist,
**gekennzeichnet durch**
das Entlasten an dem ersten Schlitzende und dem zweiten Schlitzende durch Vorsehen mindestens einer Entlastungseinrichtung, die mit zumindest einem Teil des Schlitzes (24) in der oberen Scheibe in Verbindung steht, so dass das Septum (22) eine Struktur aufweist, die gegen Reißen fest ist.

12. Verfahren nach Anspruch 11, ferner mit dem Erweitern der Schlitzbreite auf die Breite des Septums (22).

13. Verfahren nach Anspruch 11, ferner mit dem Verändern der Tiefe des Schlitzes (24) in dem Septum (22).

14. Verfahren nach Anspruch 11, ferner mit dem Drücken des Schlitzes (24) in Richtung einer geschlossenen Position in dem Körper (20).

## Revendications

1. Dispositif d'accès vasculaire, comprenant :
un corps (20) définissant une lumière (36) qui s'étend à travers le corps (20) ; et
un septum (22) au moins partiellement disposé dans le corps (20) pour au moins essentiellement fermer hermétiquement la lumière (36) qui s'étend à travers le corps (20) ;
où le septum (22) possède un axe longitudinal ;
où le septum (22) possède une fente (24) adaptée pour permettre un passage sélectif à travers le septum (22) ;
où la fente (24) possède une largeur de fente comprise entre une première extrémité de fente et une deuxième extrémité de fente ;
et
où la largeur de fente s'étend de manière orthogonale par rapport à l'axe longitudinal ;
**caractérisé en ce que** le septum (22) comprend un disque supérieur (46) et
au moins une partie de la fente au sein du disque supérieur possède au moins un dégagement, de sorte que le septum comprenne une structure qui résiste aux déchirures à proximité de la fente (24).

2. Dispositif d'accès vasculaire selon la revendication 1, dans lequel le disque supérieur (46) comprend une première section transversale et une deuxième section transversale, où la première section transversale est parallèle à la fente et est plus fine que la deuxième section transversale.

3. Dispositif d'accès vasculaire selon la revendication 1, dans lequel le au moins un dégagement est aligné avec la largeur de fente et dans lequel de préférence au moins une partie du au moins un dégagement est perpendiculaire à la largeur de fente.

4. Dispositif d'accès vasculaire selon la revendication 1, dans lequel la première extrémité de fente et la deuxième extrémité de fente se terminent chacune au niveau d'un trou (86) au sein du disque supérieur (46).

5. Dispositif d'accès vasculaire selon la revendication 1, dans lequel la première extrémité de fente (88) et la deuxième extrémité de fente (88) s'étendent dans une direction opposée à la direction dans laquelle tournerait un dispositif d'accès vasculaire distinct lors de l'accès au septum (22).

6. Dispositif d'accès vasculaire selon la revendication 1, dans lequel la largeur de fente s'étend sur toute la largeur du septum (22) au niveau du disque supérieur (46).

7. Dispositif d'accès vasculaire selon la revendication 1, qui comprend en outre des supports entre le corps (20) et le septum (22), où les supports appliquent une force de compression contre la fente (24) afin d'encourager la fente (24) vers une position fermée.

8. Dispositif d'accès vasculaire selon la revendication 6, dans lequel la largeur de fente s'étend sur toute la largeur du septum (22) le long de l'intégralité du septum (22), et dans lequel la largeur de fente forme un trajet irrégulier entre la première extrémité de fente et la deuxième extrémité de fente.

9. Dispositif d'accès vasculaire selon la revendication 8, dans lequel le septum (22) est formé de deux côtés essentiellement identiques.

10. Dispositif d'accès vasculaire selon la revendication 8, dans lequel le diamètre externe des deux côtés, lorsqu'ils sont combinés, est supérieur au diamètre interne du corps (20) où le septum (22) est disposé.

11. Procédé de fabrication d'un dispositif d'accès vasculaire, comprenant les étapes suivantes :
mettre à disposition un corps (20) ayant une première région d'extrémité de corps et une deuxième région d'extrémité de corps, et définissant un passage qui s'étend à travers les deux régions d'extrémité de corps ;
mettre à disposition un septum (22), où le septum comprend une première région d'extrémité de septum et une deuxième région d'extrémité de septum, où le septum comprend en outre une fente (24) qui s'étend à partir de la première région d'extrémité de septum jusqu'à la deuxième région d'extrémité de septum,
disposer au moins une partie du septum (22) dans le corps (20) pour au moins essentiellement hermétiquement fermer le passage qui s'étend à travers le corps (20), où la fente (24) du septum (22) est adaptée pour permettre un passage sélectif à travers le septum et le corps (20) ; et
où le septum (22) possède un axe longitudinal ; où la fente (24) possède une largeur de fente comprise entre une première extrémité de fente et une deuxième extrémité de fente ; où la largeur de fente s'étend de manière orthogonale par rapport à l'axe longitudinal ; et où le septum (22) comprend un disque supérieur,
**caractérisé par**
le dégagement d'une contrainte au niveau de la première extrémité de fente et de la deuxième extrémité de fente en mettant à disposition au moins une formation de dégagement en communication avec au moins une partie de la fente (24) au sein du disque supérieur de sorte que le septum (22) comprenne une structure qui résiste aux déchirures.

12. Procédé selon la revendication 11, qui comprend en outre l'étape d'étendre la largeur de fente à la largueur du septum (22).

13. Procédé selon la revendication 11, qui comprend en outre l'étape de faire varier la profondeur de la fente (24) au sein du septum (22).

14. Procédé selon la revendication 11, qui comprend en outre l'étape de comprimer la fente (24) vers une position fermée au sein du corps (20).
